# EUROPEAN PATENT SPECIFICATION

(11) **EP 3 344 768 B1**
(45) Date of publication and mention of the grant of the patent: **25.12.2019**
(21) Application number: 16753667.1
(22) Date of filing: 18.08.2016
(51) Int. Cl.: C12N 15/113, A61K 31/7105, A61P 9/10

(54) **MICRORNAS FOR THE TREATMENT OF HEART DISEASES**
MICRORNAS ZUR BEHANDLUNG VON HERZERKRANKUNGEN
MICROARN POUR LE TRAITEMENT DE CARDIOPATHIES

(30) Priority: 01.09.2015 EP 15183318
(43) Date of publication of application: 11.07.2018
(73) Proprietor: Universiteit Maastricht, 6211 LK Maastricht (NL); Academisch Ziekenhuis Maastricht, 6229 HX Maastricht (NL)
(72) Inventor: WINDT, DE, Leon Johannes, 6215 JJ Maastricht (NL); DIRKX, Ellen, 6229 ER Maastricht (NL); GIACCA, Mauro, 34149 Trieste (IT)
(74) Representative: Algemeen Octrooi- en Merkenbureau B.V.
(86) International application number: PCT/EP2016/069636
(87) International publication number: WO 2017/036811

(56) References cited:
- WO-A1-2010/135570
- WO-A1-2013/048734
- WO-A1-2014/028762
- WO-A1-2014/099999
- WO-A2-2009/137807
- Zhihua Liu ET AL: "MiR-106b and MiR-15b Modulate Apoptosis and Angiogenesis in Myocardial Infarction", Cellular Physiology and Biochemistry, 11 May 2012 (2012-05-11), pages 851-862, XP055248837, Basel, Freiburg, Paris, London, New York, New Delhi, Bangkok, Singapore, Tokyo, Sydney DOI: 10.1159/000258197 Retrieved from the Internet: URL:http://www.karger.com/Article/Pdf/2581 97 [retrieved on 2016-02-09]
- J. SEMO ET AL: "The 106b 25 microRNA cluster is essential for neovascularization after hindlimb ischaemia in mice", EUROPEAN HEART JOURNAL, vol. 35, no. 45, 17 February 2013 (2013-02-17), pages 3212-3223, XP055248886, GB ISSN: 0195-668X, DOI: 10.1093/eurheartj/eht041
- CHRISTINE WAHLQUIST ET AL: "Inhibition of miR-25 improves cardiac contractility in the failing heart", NATURE, vol. 508, no. 7497, 12 March 2014 (2014-03-12), pages 531-535, XP055248889, United Kingdom ISSN: 0028-0836, DOI: 10.1038/nature13073
- ZOLTÁN V. VARGA ET AL: "MicroRNA-25-dependent up-regulation of NADPH oxidase 4 (NOX4) mediates hypercholesterolemia-induced oxidative/nitrative stress and subsequent dysfunction in the heart", JOURNAL OF MOLECULAR AND CELLULAR CARDIOLOGY., vol. 62, 27 May 2013 (2013-05-27), pages 111-121, XP055248884, GB ISSN: 0022-2828, DOI: 10.1016/j.yjmcc.2013.05.009
- ELLEN DIRKX ET AL: "Nfat and miR-25 cooperate to reactivate the transcription factor Hand2 in heart failure", NATURE CELL BIOLOGY, vol. 15, no. 11, 27 October 2013 (2013-10-27), pages 1282-1293, XP055248935, GB ISSN: 1465-7392, DOI: 10.1038/ncb2866
- LEI PAN ET AL: "MiR-25 Protects Cardiomyocytes against Oxidative Damage by Targeting the Mitochondrial Calcium Uniporter", INTERNATIONAL JOURNAL OF MOLECULAR SCIENCES, vol. 16, no. 3, 10 March 2015 (2015-03-10) , pages 5420-5433, XP055248943, DOI: 10.3390/ijms16035420

## Description

### Field of the invention

The invention relates to the field of molecular biology and medicine, more specifically the invention is directed towards the treatment, delay and amelioration of heart diseases. More in particular, microRNAs are provided that induce cardiac regeneration by inducing cell cycle proliferation of cardiomyocytes, thereby treating or ameliorating heart diseases associated with a loss of cardiomyocytes or cardiomyocyte function. Such diseases include myocardial infarction, cardiomyopathy of ischemic or non-ischemic origin, myocarditis and heart failure.

### Background of the invention

Cardiovascular diseases, including hypertension, coronary artery disease and genetic forms of cardiomyopathies may result in heart failure, which is associated with pathological remodelling of the myocardium, pump failure and sudden death. Epidemiological analysis in Western countries indicates that cardiovascular disorders are among the first causes of morbidity and mortality among people over 60 years. There are approximately 600,000 deaths per year in Europe from myocardial infarction and, even more relevant, heart failure is estimated to affect over 15 million people worldwide, representing one of the leading causes of death. This number is likely going to increase as a consequence of the ageing of the global population. While conventional pharmacological treatment strategies (e.g., β-blockers and ACE-inhibitors) have shown effectiveness in prolonging survival of heart failure patients [1], the prognosis of affected individuals remains poor, leaving a need for new concepts.

In mammals, enlargement of the heart during embryonic development is primarily dependent on the increase in cardiomyocyte number, but early after birth cardiac myocytes stop proliferating and further growth of the myocardium occurs through hypertrophic enlargement of existing myocardial cells [2]. In the mouse, the stop in cell cycle activity of heart muscle cells occurs shortly after birth; whereas human cardiomyocytes show a striking reduction in the proliferative capacity after 7 months of age. Recent evidences obtained by dating of cardiomyocyte DNA in humans has indicated that cardiomyocytes physiologically renew at a rate of 1% at the age of 25 and 0.45% at the age of 75, and that fewer than 50% of the cardiomyocytes are exchanged during the normal life span [3].

As a consequence of this limited proliferation capacity of adult cardiomyocytes, the ability of the mammalian adult heart to repair itself following injury is very restricted [4]. In particular, the loss of cardiomyocyte that occurs after various types of myocardial damage, typically after myocardial infarction, is not repaired by the generation of new contractile tissue but by the formation of a scar, which compromises cardiac function and often tends to worsen over time, leading to heart failure. Thus, the identification of novel means to promote the regeneration of contractile cardiac tissue after injury appears mandatory. However, the exact molecular mechanisms controlling the cell cycle in the adult cardiomyocyte remain largely unknown.

MicroRNAs (MiRs) are a family of small (19-25 nucleotide) single-stranded non-coding RNA molecules that regulate gene expression at the post-transcriptional level. Inhibition of gene expression occurs through complementary base pairing with sequences mainly located in the 3' untranslated region (3' UTR) of the target mRNA [5], leading to translational repression or mRNA degradation. Key recognition elements comprise nucleotides 2-8 at the 5' end of the microRNA and are known as seed sequences [6]. MicroRNAs are often represented as families, defined by conservation of their seed region, with conservation of sequences from nematodes through to humans, implying importance of function during evolution. Between 10-40% of human mRNAs are regulated by microRNAs whereby single microRNA species can regulate multiple mRNA targets and single microRNAs may contain several microRNA recognition sites in their 3'UTR [7]. Such complex regulatory networks can control key biological functions and alterations in microRNA expression are associated with numerous human pathologies including cardiovascular diseases [8, 9].

In contrast to many cellular factors involved in disease, which are difficult to modulate therapeutically, microRNA levels can be easily modulated in vivo by using microRNA mimics which provide a surrogate microRNA action and antimiRs which are RNA molecules comprising sequences complementary to the mature microRNA sequence. Through this complementarity, the antimiRs hybridize to the microRNAs and thereby block their activity. In fact, the efficient use of antimiRs has been demonstrated in non-human primates [10, 11], and these studies have been advanced to human clinical trials [12].

Only a few microRNAs have been so far clearly implicated in cardiomyocyte proliferation, including miR-1, miR-133, miR-199a, miR-590 and members of the miR-15 family. Overexpression of miR-1 in the embryonic heart was shown to inhibit cardiomyocyte proliferation, which was linked to the repression of Hand2, a transcription factor required for cardiac growth during embryogenesis [13]. miR-133 inhibits proliferation of cardiomyocytes through the repression of SRF and cyclin D2, two essential regulators of muscle cell differentiation [14]. The miR-15 family was shown to regulate the post-natal mitotic arrest of mouse cardiomyocytes, through the downregulation of the expression of Chekl [15]. Exogenous administration of miR-590 and miR-199a) were shown to promote cell cycle re-entry of adult cardiomyocytes ex vivo [16].

Despite the above detailed advances, there remains a need in the art for better and more effective and efficient treatments of heart diseases.

### Summary of the invention

The invention provides a composition comprising microRNA 106b for use in the treatment of a heart disease wherein the treatment comprises the induction of cardiomyocyte cell cycle proliferation and wherein the treatment is performed after a heart infarct.

### Legend to the figures

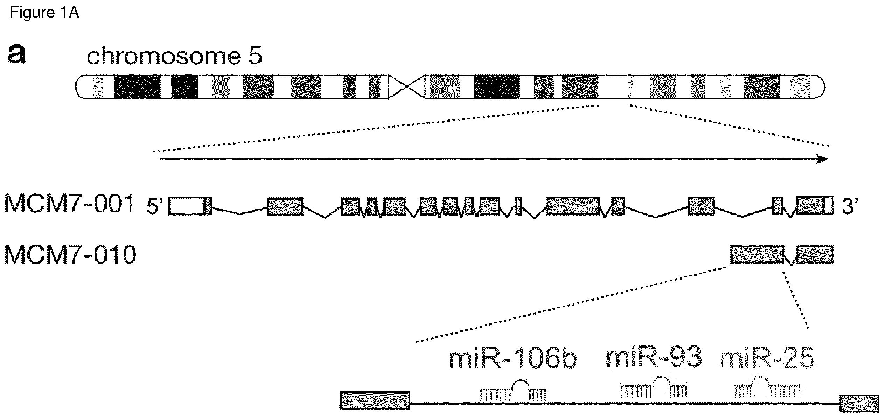

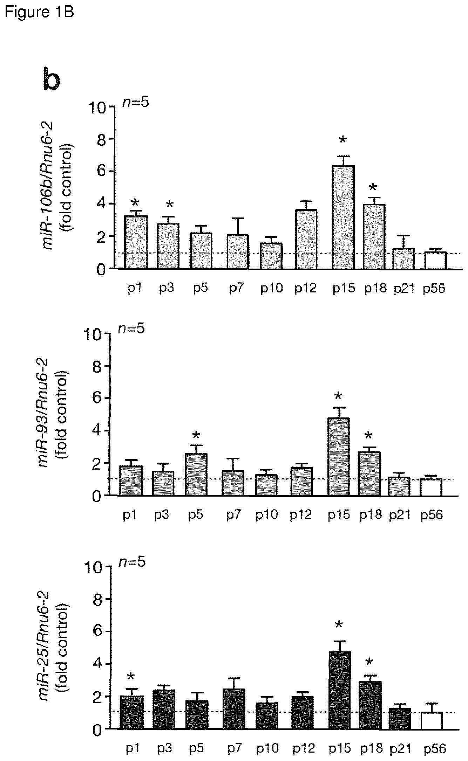

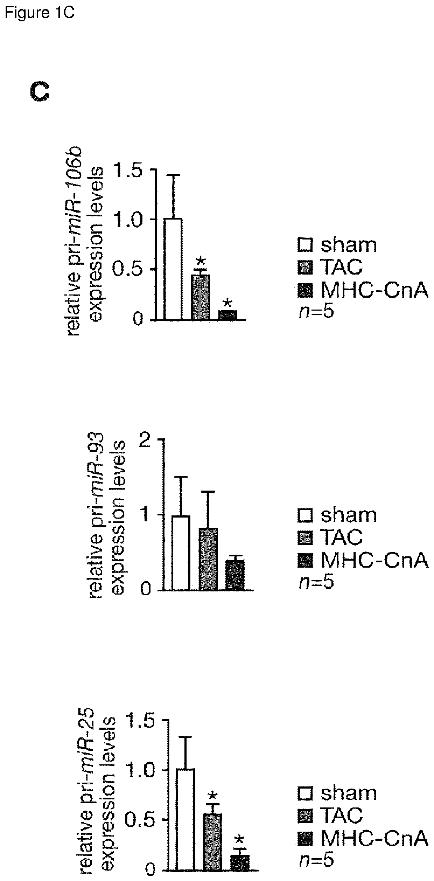

Figure 1. Genomic localization of the MicroRNA-106b∼25 cluster.
   Panel (a) The miR-106b∼25 microRNA cluster is located within the minichromosome maintenance deficient 7 (Mcm7) gene on chromosome 5.
   Panel (b) Graph showing the expression levels of individual members of the miR-106b∼25 microRNA cluster, miR-106b, miR-93 and miR-25 in hearts from mice at distinct time points after birth. P1 indicates I week after birth, P2 two weeks etcetera
   Panel (c) Graph showing the expression levels of individual members of the miR-106b∼25 microRNA cluster, miR-106b, miR-93 and miR-25 in adult hearts from mice under control conditions, after transverse aortic constriction (TAC) or from transgenic mice with cardiac-specific overexpression of calcineurin (MHC-CnA).

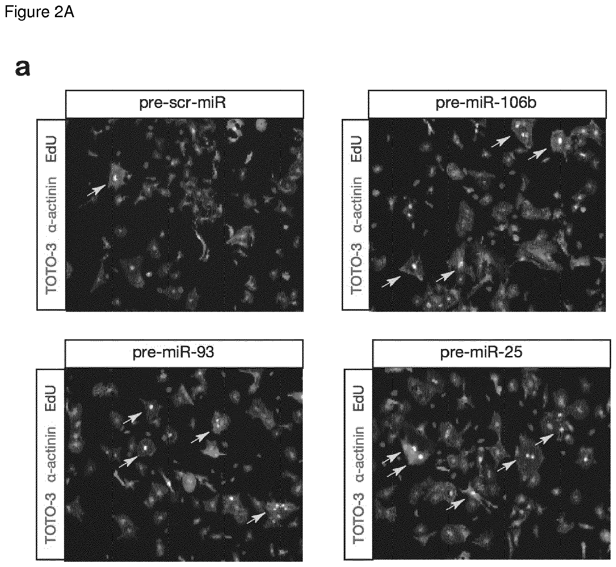

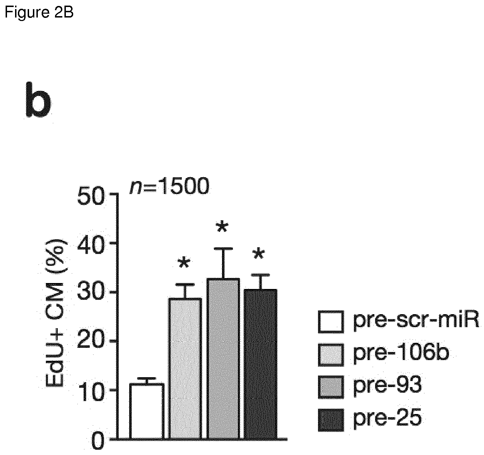

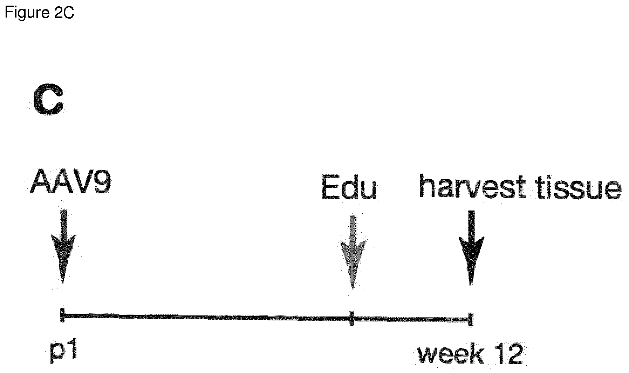

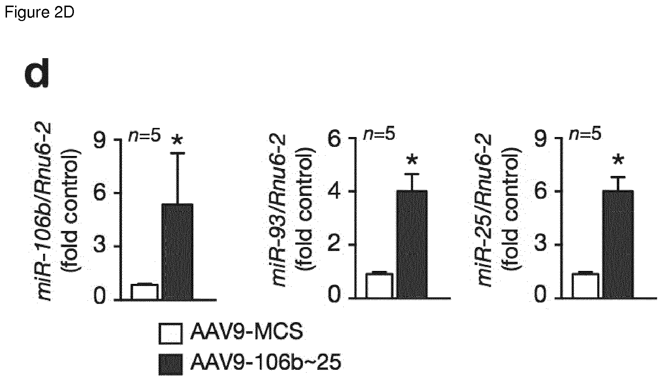

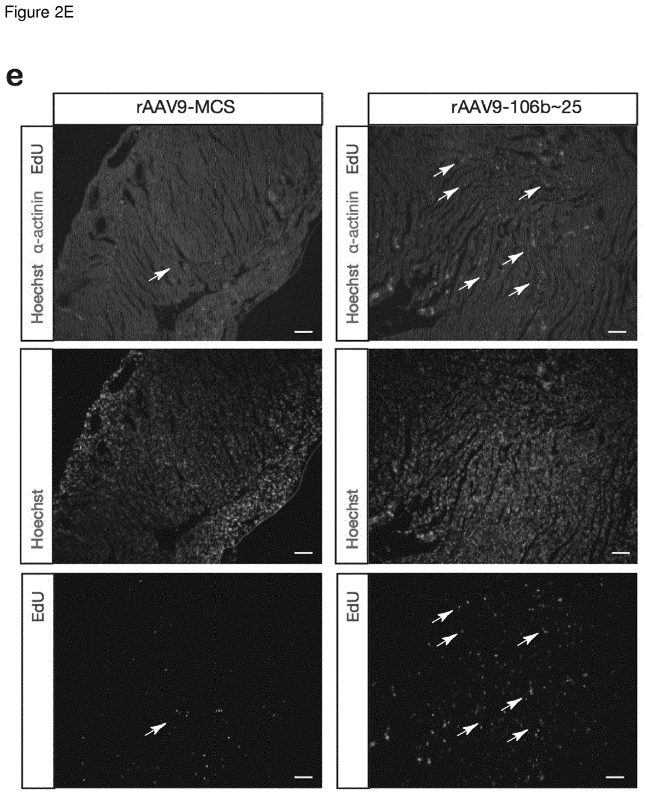

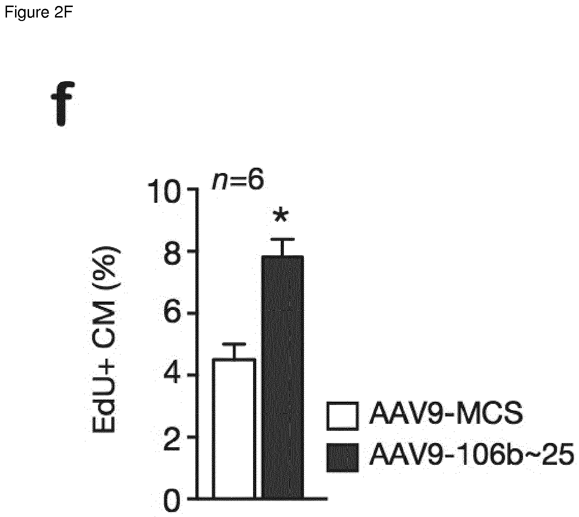

Figure 2. Overexpression of the MicroRNA-106b∼25 cluster induces cardiomyocyte proliferation in vitro and in vivo.
   Panel (a) Overexpression of the miR-106b∼25 microRNA cluster in cultured cardiomyocytes by transfection with precursor molecules for each cluster member results in proliferation as measured by Edu (5-ethynyl-2'-deoxyuridine) incorporation.
   Panel (b) Graph showing the quantification of the number of proliferating, cultured cardiomyocytes.
   Panel (c) Graph showing the set-up of the experiment. Ten days before sacrifice, the animals received an injection with Edu to mark proliferating cells in vivo. Twelve weeks after the injection, the adult hearts were removed for further analysis.
   Panel (d) Graph showing the overexpression of the miR-106b∼25 microRNA cluster in vivo by adeno-associated virus 9 (AAV9) gene therapy resulting in proliferation as measured by Edu incorporation.
   Panel (e) Quantification of the number of proliferating cardiomyocytes in vivo. Confocal microscopy of the free left ventricular wall stained for alpha-actinin to identify cardiac muscle, Hoechst to identify nuclei and Edu for proliferating myocytes.
   Panel (f) Graph showing that EdU positive cardiomyocytes following AAV9-106b∼25 gene therapy demonstrated an approximate doubling of the number of adult proliferating cardiomyocytes as compared to AAV9-MCS therapy (EdU+ CM%).

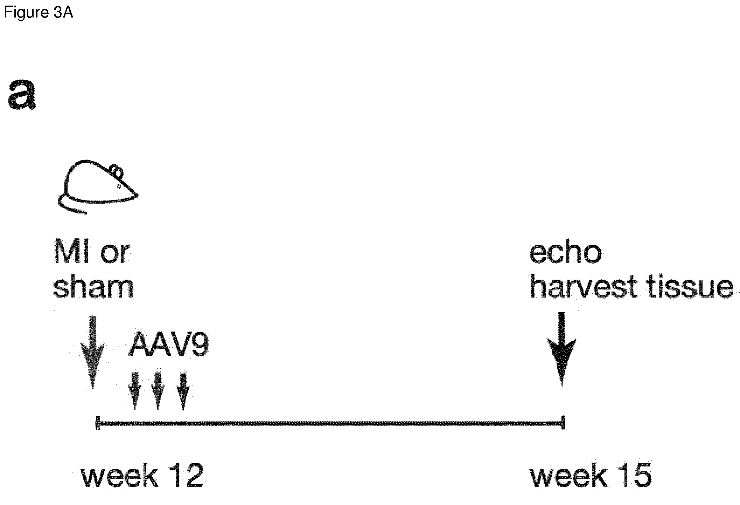

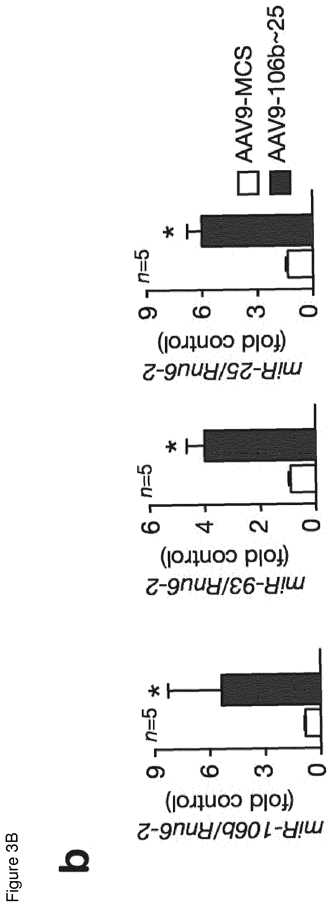

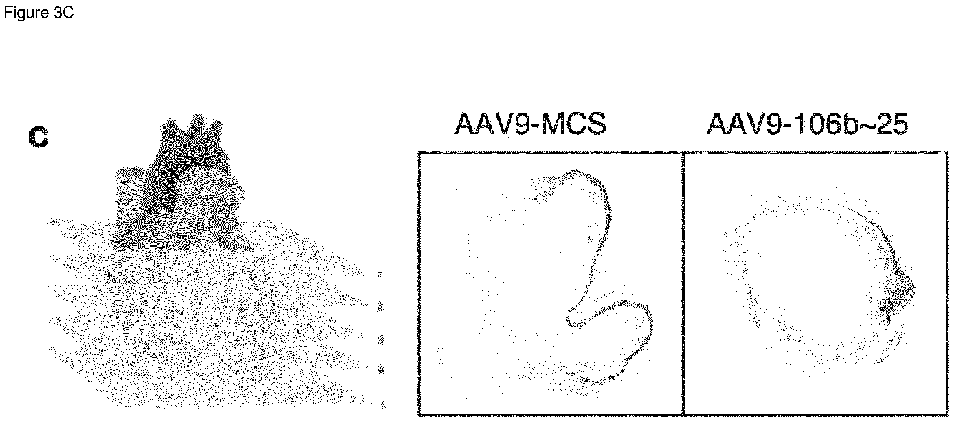

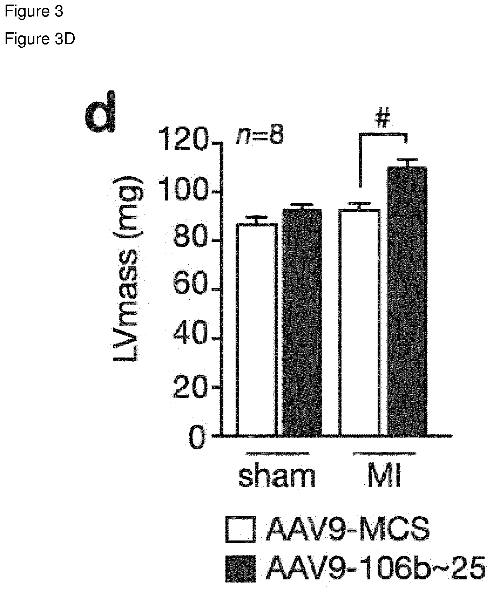

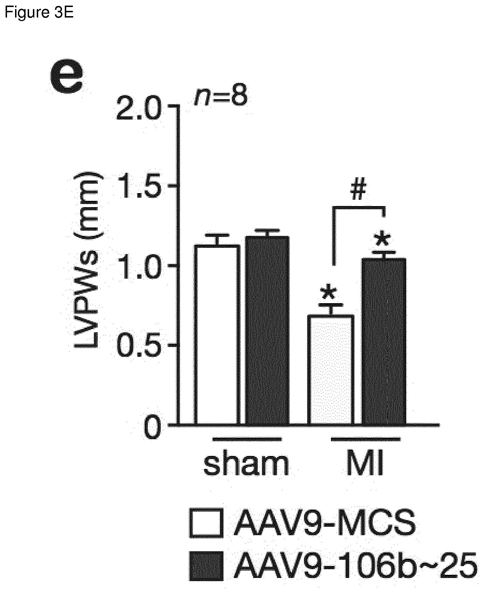

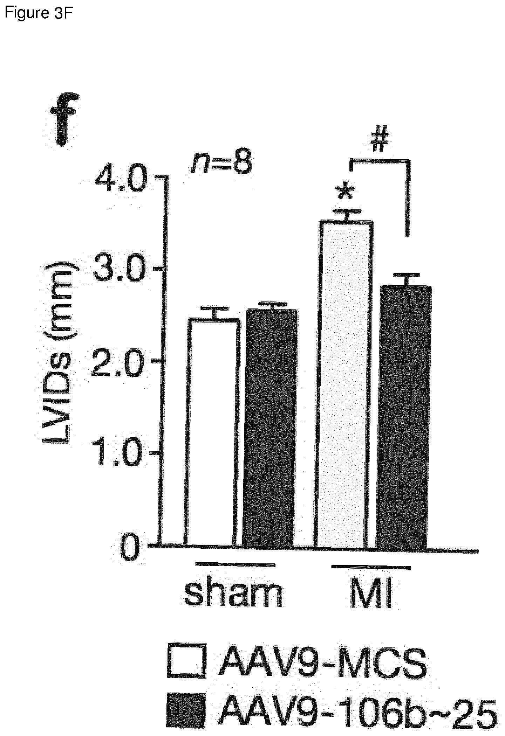

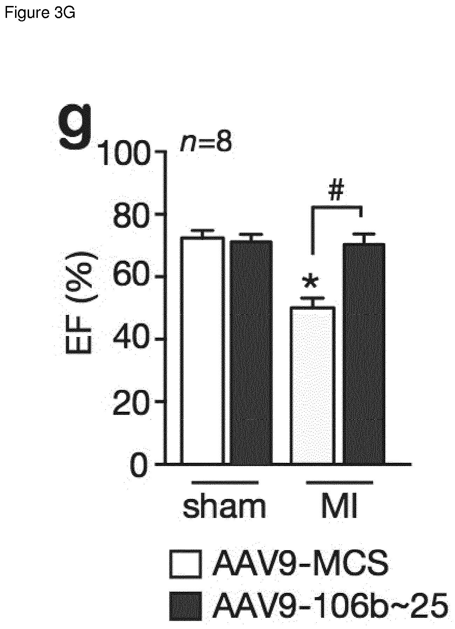

Figure 3. MicroRNA-106b∼25 gene therapy results in regeneration of the infarcted heart in vivo.
   Panel (a) Schematic representation of the study setup. Mice were randomized to receive a sham surgery or myocardial infarction (MI). Immediately after the infarct, animals received either a control AAV9 (AAV9-MCS) or AAV9-106b∼25, designed to overexpress the microRNA-106b∼25 cluster members.
   Panel (b) demonstration that AAV9-106b∼25 increases myocardial expression of miR-106b, miR-93 and miR-25.
   Panel (c) Schematic representation of the plane of section through the infarcted heart. Sirius red staining indicates a large infarct (red staining) in infarcted animals that received AAV9-MCS gene therapy, and a substantially reduced infarct size in infarcted animals that received AAV9-106b∼25 gene therapy.
   Panel (d) Infarcted animals that received AAV9-106b∼25 gene therapy have greatly increased left ventricular mass.
   Panel (e) Infarcted animals that received AAV9-106b∼25 gene therapy have maintained left ventricular posterior wall thickness.
   Panel (f) Infarcted animals that received AAV9-106b∼25 gene therapy have reduced left ventricular internal dimension.
   Panel (g) Infarcted animals that received AAV9-106b∼25 gene therapy have maintained ejection fraction.

### Detailed description

The invention provides a composition comprising microRNA 106b for use in the treatment of a heart disease wherein the treatment comprises the induction of cardiomyocyte cell cycle proliferation and wherein the treatment is performed after a heart infarct.

Also disclosed herein is a composition comprising a microRNA selected from the group consisting of microRNA 106, microRNA 93 or microRNA 25 and the complements thereof, for use in the treatment, prevention, delay or amelioration of a heart disease.

In other terms, the disclosure provides a method of treating, preventing, delaying, or ameliorating a heart disease wherein a composition comprising a microRNA selected from the group consisting of microRNA 106b, microRNA 93 or microRNA 25 and the complements thereof is administered to a subject in need thereof.

In mammals, during embryogenesis and early after birth, the heart retains an ability to grow through cardiac muscle proliferation, including a preadolescent burst in myocyte proliferation, shortly before adulthood. In the mouse, the stop in cell cycle activity of heart muscle cells occurs shortly after birth; whereas human cardiomyocytes show a striking reduction in the proliferative capacity after 7 months of age. Recent evidences obtained by dating of cardiomyocyte DNA in humans has indicated that cardiomyocytes physiologically renew at a rate of 1% at the age of 25 and 0.45% at the age of 75, and that fewer than 50% of the cardiomyocytes are exchanged during the normal life span [3]. As a consequence of this limited proliferation capacity of adult cardiomyocytes, the ability of the mammalian adult heart to repair itself following myocardial infarction is very restricted [4], tissue injury is not repaired by the generation of new contractile tissue but by the formation of a scar, which compromises cardiac function and often tends to worsen over time, leading to heart failure. Thus, the identification of novel means to promote cell cycle reactivation in adult cardiac myocytes may have regenerative consequences for the myocardium.

We discovered that microRNAs 160b, microRNA 93 and microRNA 25, herein together referred to as the microRNA cluster miR-106b∼25, display high expression in early stages after birth and low expression in the adult heart. The intronic miR-106b∼25 cluster is part of the minichromosome maintenance deficient 7 (Mcm7) gene and located in mice on chromosome 5 and in human on chromosome 7 in the same genetic organization (Figure 1a).

The miR-106b∼25 cluster harbors 3 microRNAs, miR-106b, miR-93 and miR-25 and are co-transcribed. First, we tested the expression of miR-106b, miR-93 and miR-25 in 5 hearts from wildtype mice sacrificed at postnatal (P) day 1 (P1), P3, P5, P7 (or 1 week after birth), P10, P12, P15, P18, P21 (or 3 weeks after birth) and at P56 (or 8 weeks after birth; Figure 1b). The data show that compared to adult hearts (P56), miR-106b, miR-93 and miR-25 display an expression level of at least 2 fold higher than adult expression levels with a characteristic additional elevation between 5-7 fold around P15. This characteristic additional elevation corresponds to a recently described brief but intense proliferative burst of predominantly binuclear cardiomyocytes in preadolescent hearts in mice and humans [19].

Finally, we also evaluated expression of miR-106b, miR-93 and miR-25 in healthy adult myocardium compared to adult heart failure myocardium resulting from sustained pressure overloading by transverse aortic constriction (TAC) or from transgene expressing a constitutively activated form of the pro-hypertrophic phosphatase calcineurin in the myocardium (MHC-CnA). In line with the very limited ability of the mammalian adult heart to induce cardiomyocyte proliferation and to repair itself, the expression of miR-106b, miR-93 and miR-25 was even lower in failing mouse hearts compared to the healthy myocardium. In conclusion, these data show that members of the microRNA cluster miR-106b∼25 are elevated in expression in the myocardium at time points that correlate with activity of the cell cycle and proliferation of cardiac muscle cells.

Next, to evaluate whether the increased expression of the microRNA cluster miR-106b∼25 would result in re-entry of the cell cycle, we cultured neonatal rat cardiomyocytes and stimulated the expression of either miR-106b, miR-93 or miR-25 by precursor microRNA transfection for 24 hrs. Following fixation, we treated the cultures with an antibody for alpha-actinin to detect individual myocytes, counterstained the nuclei with the nuclear stain TOTO-3 and using the nucleoside analogue EdU (5-ethynyl-2'-deoxyuridine) for thymidine substitution to detect chromosomal DNA synthesis characteristic of the S1 cell cycle phase.

Only myocytes with EdU positivity were considered in the proliferation assay and 1500 cell where counted in each condition (Figure 2a). The data demonstrate that cultured myocytes transfected with a control precursor for scrambled microRNA (pre-scr-miR) demonstrated that 10% of myocytes were proliferating. In contrast, transfection with either miR-106b, miR-93 or miR-25 elevated the proliferation rate of myocytes to around 30% without any apparent difference between the individual members of the microRNA cluster miR-106b-25 (Figure 2b).

To verify these results in vivo, we induced elevated expression of miR-106b, miR-93 and miR-25 by construction of a recombinant adeno-associated virus engineered to overexpress the microRNA cluster miR-106b∼25 (AAV9-106b∼25). AAV9 has the advantage that it has a natural cardiac tropism for the myocardium resulting in myocardial restricted gene therapy characteristics when injected in animals or humans. To accomplish cardiac gene therapy with overexpression of miR-106b, miR-93 and miR-25, mice were injected at P1 with either 10¹¹ particles of a control AAV9 with only the empty multiple cloning site (AAV9-MCS) or AAV9-106b∼25 in the tail vein. Ten days before sacrifice, the animals received an injection with Edu to mark proliferating cells in vivo. Twelve weeks after the injection, the adult hearts were removed for further analysis (Figure 2c). The data show that miR-106b, miR-93 and miR-25 were elevated between 4 and 6 fold in mice that received AAV9-106b∼25 gene therapy compared to gene therapy with the control AAV9-MCS vector (Figure 2d). Confocal microscopy of the free left ventricular wall stained for alpha-actinin to identify cardiac muscle, Hoechst to identify nuclei and Edu for proliferating myocytes demonstrated a dramatic elevation of myocyte proliferation in vivo following AAV9-106b∼25 gene therapy (Figure 2e). Quantification of EdU positive cardiomyocytes following either AAV9-MCS or AAV9-106b∼25 gene therapy demonstrated an approximate doubling of the number of adult proliferating cardiomyocytes (EdU+ CM; Figure 2f).

In conclusion, the combined data demonstrate that elevation of miR-106b, miR-93 and miR-25 evoked cell cycle re-entry and proliferation of cardiomyocytes both in short term cultures in vitro as well as proliferation of cardiomyocytes in the adult myocardium in vivo.

Next we assessed whether elevated expression of miR-106b∼25 might boost the normally ineffective myocardial repair that takes place after myocardial infarction. Adult CD1 mice (8-12-weeks old) underwent permanent left anterior descending coronary artery ligation to induce myocardial infarction (MI) or sham operation and were injected, in the peri-infarcted area, with AAV9 vectors expressing the three miRNAs (AAV9-106b∼25) or a control vector (Figure 3a). In line with the above presented results, this treatment results in efficient myocardial transduction and month-long transgene expression with 4 to 6-fold elevation of individual miRNA cluster members (Figure 3b). Sirius red staining of cross sections just below the ligation indicated a large and thinned infarct in mice subjected to MI and injected with the AAV9 control vector, while the myocardial integrity was much better preserved and infarct was much smaller in mice subjected to MI and receiving the AAV9 vector designed to overexpress the miR-106b∼25 cluster. As evaluated by echocardiography, left ventricular mass was increased in infarcted mice receiving AAV9-106∼25, indicating preserved muscularity (Figure 3d). Functionally, left ventricular posterior wall thickness (LVPW, Figure 3e), left ventricular internal diameter (LVID; Figure 3f) and left ventricular ejection fraction (EF, Figure 3g) were significantly preserved over time in the infarcted mice injected with AAV9-106b∼25.

In conclusion, these data show that the expression of miR-106b∼25 after infarction exerts a marked beneficial effect in reducing infarct size and improving cardiac function, consistent with the effect of these miRNAs in actively stimulating cardiomyocyte proliferation and regenerative capacity of the heart.

### Examples

### Example 1: Primary cardiomyocytes cultures, immunocytochemistry.

Cardiomyocyte cultures were isolated by enzymatic dissociation of 1- 2- day-old neonatal rat hearts and processed for immunofluorescence as described previously [17]. Neonatal cardiomyocytes were transfected with precursors (Ambion) of microRNAs (10mM) using Oligofectamin (Invitrogen). For visualization of cardiomyocyte size and sarcomeric organization the cells were stained for α2-actinin with mouse monoclonal anti-sarcomeric α-actinin antibody (Sigma-Aldrich, A7811 clone EA-53, 1:500) followed by rat anti-mouse monoclonal antibody Oregon green-conjugated antibody (Life Technologies, A-889, 1:1000). Nuclear staining was performed with VECTASHIELD Mounting Medium (Vector Laboratories) with 4',6-diamidino-2-phenylindole (DAPI).

### Example 2: Luciferase 3'-UTR reporter assays

Constructs bearing the murine 3'-UTR of Cdkn1a, Cdkn1c or E2f5 were obtained PCR amplification and subcloned into psiCHECK2 vector (Promega). HL-1 cells were transfected with a scrambled precursor or precursor for mmu-miR1-106b, mmu-miR-93 or mmu-miR-25 (Exiqon) at a final concentration of 10 nM in 48-well plates using oligofectamine (Invitrogen). The luciferase reporters were transfected using Fugene-6 reagent (Roche). Twenty-four hours after transfection of the reporters and miRNAs, Firefly and Renilla luciferase activities were measured 48 h after plasmid transfection using the Dual Luciferase Reporter Assay System (Promega), according to the manufacturer's instructions.

### Example 3: Production and purification of recombinant AAV vectors

The murine miR-106b∼25 cluster plus upstream and downstream flanking sequences (total approximating 300 base pairs) were amplified from human genomic DNA isolated from HeLa cells, using the QIAamp DNA mini kit (Qiagen), according to the manufacturer's instructions. The amplified sequences were cloned into the pZac2.1 vector (Gene Therapy Program, Penn Vector core, University of Pennsylvania, USA), which was used to produce recombinant AAV vectors. Recombinant AAV vectors were prepared in the AAV Vector Unit at ICGEB Trieste, as described previously [18]. Briefly, AAV vectors of serotype 9 were generated in HEK293T cells, using a triple-plasmid co-transfection for packaging. Viral stocks were obtained by CsCl₂ gradient centrifugation. Titration of AAV viral particles was performed by real-time PCR quantification of the number of viral genomes, as described previously; the viral preparations had titres between 1 × 10¹³ and 3 × 10¹³ viral genome particles per ml.

### Example 4: Injection of AAV vectors in neonatal and adult mice

In the experiments using AAV vectors, neonatal CD1 mice (post-natal day 1) were intra-peritoneally injected with AAV9-MCS (AAV9-control) or AAV9-106b∼25 at a dose of 1 × 10¹¹ viral genome particles per animal, using an insulin syringe with incorporated 30-gauge needle. The hearts of the injected mice were collected 12 weeks after AAV injection.

Adult mice intracardiac injection of AAV vectors (AAV9-control or AAV9-106b∼25), at a dose of 1 × 10¹¹ viral genome particles per animal, was performed as described below for animals that underwent myocardial infarction. The hearts of the injected animals were collected 12 days after AAV injection.

### Example 5: Myocardial infarction

Myocardial infarction was produced in adult CD1 mice (12 weeks old), by permanent left anterior descending (LAD) coronary artery ligation. Briefly, mice were anesthetized with an intraperitoneally injection of ketamine and xylazine, endotracheally intubated and placed on a rodent ventilator. Body temperature was maintained at 37 °C on a heating pad. The beating heart was accessed via a left thoracotomy. After removing the pericardium, a descending branch of the LAD coronary artery was visualized with a stereomicroscope (Leica) and occluded with a nylon suture. Ligation was confirmed by the whitening of a region of the left ventricle, immediately post-ligation. Recombinant AAV vectors, at a dose of 1 × 10¹¹ viral genome particles per animal, were injected immediately after LAD ligation into the myocardium bordering the infarct zone (single injection), using an insulin syringe with incorporated 30-gauge needle.

### Example 6: Echocardiography analysis

To evaluate left ventricular function and dimensions, transthoracic two-dimensional echocardiography was performed on mice sedated with 5% isoflurane at 12, 30 and 60 days after myocardial infarction, using a Visual Sonics Vevo 770 Ultrasound (Visual Sonics) equipped with a 30-MHz linear array transducer. M-mode tracings in parasternal short axis view were used to measure left ventricular anterior and posterior wall thickness and left ventricular internal diameter at end-systole and end-diastole, which were used to calculate left ventricular fractional shortening and ejection fraction.

### Example 7: Heart collection and histological analysis

At the end of the studies, animals were anaesthetized with 5% isoflurane and then killed by injection of 10% KCI, to stop the heart at diastole. The heart was excised, briefly washed in PBS, weighted, fixed in 10% formalin at room temperature, embedded in paraffin and further processed for histology or immunofluorescence. Haematoxylin-eosin and Sirius red staining were performed according to standard procedures, and analysed for regular morphology and extent of fibrosis.

### References.

1. Benjamin IJ, Schneider MD. Learning from failure: congestive heart failure in the postgenomic age. J Clin Invest. 2005;115:495-9.
2. Ahuja P, Sdek P, MacLellan WR. Cardiac myocyte cell cycle control in development, disease, and regeneration. Physiol Rev. 2007;87:521-44.
3. Bergmann O, Bhardwaj RD, Bernard S, Zdunek S, Barnabe-Heider F, Walsh S, et al. Evidence for cardiomyocyte renewal in humans. Science. 2009;324:98-102.
4. Senyo SE, Steinhauser ML, Pizzimenti CL, Yang VK, Cai L, Wang M, et al. Mammalian heart renewal by pre-existing cardiomyocytes. Nature. 2013;493:433-6.
5. Ambros V. The functions of animal microRNAs. Nature. 2004;431:350-5.
6. Kim VN. MicroRNA biogenesis: coordinated cropping and dicing. Nat Rev Mol Cell Biol. 2005;6:376-85.
7. Bartel DP. MicroRNAs: target recognition and regulatory functions. Cell. 2009; 136:215-33.
8. Olson EN. MicroRNAs as Therapeutic Targets and Biomarkers of Cardiovascular Disease. Sci Transl Med. 2014;6:239ps3.
9. Da Costa Martins PA, De Windt LJ. MicroRNAs in control of cardiac hypertrophy. Cardiovasc Res. 2012;93:563-72.
10. Weiler J, Hunziker J, Hall J. Anti-miRNA oligonucleotides (AMOs): ammunition to target miRNAs implicated in human disease? Gene Ther. 2006;13:496-502.
11. Elmen J, Lindow M, Schutz S, Lawrence M, Petri A, Obad S, et al. LNA-mediated microRNA silencing in non-human primates. Nature. 2008;452:896-9.
12. Janssen HL, Reesink HW, Lawitz EJ, Zeuzem S, Rodriguez-Torres M, Patel K, et al. Treatment of HCV infection by targeting microRNA. N Engl J Med. 2013;368:1685-94.
13. Zhao Y, Samal E, Srivastava D. Serum response factor regulates a muscle-specific microRNA that targets Hand2 during cardiogenesis. Nature. 2005;436:214-20.
14. Liu N, Bezprozvannaya S, Williams AH, Qi X, Richardson JA, Bassel-Duby R, et al. microRNA-133a regulates cardiomyocyte proliferation and suppresses smooth muscle gene expression in the heart. Genes Dev. 2008;22:3242-54.
15. Porrello ER, Johnson BA, Aurora AB, Simpson E, Nam YJ, Matkovich SJ, et al. MiR-15 family regulates postnatal mitotic arrest of cardiomyocytes. Circ Res. 2011; 109:670-9.
16. Eulalio A, Mano M, Dal Ferro M, Zentilin L, Sinagra G, Zacchigna S, et al. Functional screening identifies miRNAs inducing cardiac regeneration. Nature. 2012;492:376-81.
17. De Windt LJ, Lim HW, Haq S, Force T, Molkentin JD. Calcineurin promotes protein kinase C and c-Jun NH2-terminal kinase activation in the heart. Cross-talk between cardiac hypertrophic signaling pathways. J Biol Chem. 2000;275:13571-9.
18. Arsic N, Zentilin L, Zacchigna S, Santoro D, Stanta G, Salvi A, et al. Induction of functional neovascularization by combined VEGF and angiopoietin-1 gene transfer using AAV vectors. Mol Ther. 2003;7:450-9.
19. Naqvi N, Li M, Calvert JW, Tejada T, Lambert JP, Wu J, Kesteven SH, Holman SR, Matsuda T, Lovelock JD, Howard WW, lismaa SE, Chan AY, Crawford BH, Wagner MB, Martin DI, Lefer DJ, Graham RM, Husain A. A proliferative burst during preadolescence establishes the final cardiomyocyte number. Cell 2014:157; 795-807.

## Claims

1. A composition comprising microRNA 106b for use in the treatment of a heart disease wherein the treatment comprises the induction of cardiomyocyte cell cycle proliferation and wherein the treatment is performed after a heart infarct.

2. The composition for use according to claim 1 wherein the heart disease is coronary artery disease.

3. The composition for use according to claim 1 or 2 wherein the treatment exerts a beneficial effect in reducing infarct size.

4. The composition for use according to any one of claims 1 - 3 wherein the treatment improves cardiac function.

5. The composition for use according to any one of claims 1 - 4 wherein the microRNA is administered in a targeting vector.

6. The composition for use according to claim 5 wherein the targeting vector is an adeno-associated virus vector.

7. The composition for use according to claim 6 wherein the adeno-associated virus vector is of serotype AAV1 or AAV9.

## Patentansprüche

1. Eine Zusammensetzung, umfassend microRNA 106b zur Verwendung bei der Behandlung einer Herzerkrankung, worin die Behandlung die Induktion der Kardiomyozytenzell-Zyklusproliferation umfasst und die Behandlung nach einem Herzinfarkt durchgeführt wird.

2. Die Zusammensetzung zur Verwendung nach Anspruch 1, worin die Herzkrankheit eine die Koronararterie betreffende Erkrankung ist.

3. Die Zusammensetzung zur Verwendung nach Anspruch 1 oder 2, worin die Behandlung eine positive Wirkung bei der Reduzierung der Infarktgröße ausübt.

4. Die Zusammensetzung zur Verwendung nach einem der Ansprüche 1 bis 3, worin die Behandlung die Herzfunktion verbessert.

5. Die Zusammensetzung zur Verwendung nach einem der Ansprüche 1 bis 4, worin die microRNA in einem Zielvektor verabreicht wird.

6. Die Zusammensetzung zur Verwendung nach Anspruch 5, worin der Zielvektor ein adeno-assoziierter Virusvektor ist.

7. Die Zusammensetzung zur Verwendung nach Anspruch 6, worin der adenoassoziierte Virusvektor vom Serotyp AAV1 oder AAV9 ist.

## Revendications

1. Composition comprenant un microARN 106b pour une utilisation dans le traitement d'une maladie cardiaque, où le traitement comprend l'induction de la prolifération du cycle cellulaire des cardiomyocytes et où le traitement est effectué après un infarctus cardiaque.

2. Composition pour une utilisation selon la revendication 1, dans laquelle la maladie cardiaque est une maladie coronarienne.

3. Composition pour une utilisation selon la revendication 1 ou 2, dans laquelle le traitement exerce un effet bénéfique de réduction de la taille de l'infarctus.

4. Composition pour une utilisation selon l'une quelconque des revendications 1 à 3, dans laquelle le traitement améliore la fonction cardiaque.

5. Composition pour une utilisation selon l'une quelconque des revendications 1 à 4, dans laquelle le microARN est administré dans un vecteur de ciblage.

6. Composition pour une utilisation selon la revendication 5, dans laquelle le vecteur de ciblage est un vecteur de virus adéno-associé.

7. Composition pour une utilisation selon la revendication 6, dans laquelle le vecteur de virus adéno-associé est de sérotype AAV1 ou AAV9.
